(19) Europäisches Patentamt / European Patent Office / Office européen des brevets

(11) EP 3 581 104 A1

(12) **EUROPEAN PATENT APPLICATION**

(43) Date of publication:
18.12.2019 Bulletin 2019/51

(51) Int Cl.:
A61B 5/022 (2006.01)   A61B 5/00 (2006.01)
A61B 5/021 (2006.01)   A61B 5/08 (2006.01)
A61B 5/113 (2006.01)   A61B 5/02 (2006.01)

(21) Application number: 18177438.1

(22) Date of filing: 13.06.2018

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Koninklijke Philips N.V.
5656 AE Eindhoven (NL)

(72) Inventors:
• DE GROOT, Koen
  5656 AE Eindhoven (NL)
• GELISSEN, Jos
  5656 AE Eindhoven (NL)

(74) Representative: de Haan, Poul Erik et al
Philips International B.V.
Philips Intellectual Property & Standards
High Tech Campus 5
5656 AE Eindhoven (NL)

(54) **METHOD, DEVICE AND COMPUTER PROGRAM PRODUCT FOR ESTIMATING A COMPLIANCE OF A BLOOD VESSEL IN A SUBJECT**

(57) There is provided a device for estimating a compliance of a blood vessel in a subject, the device comprising a blood volume sensor for measuring changes in blood volume in skin of the subject and for outputting a blood volume signal; an actuator arranged to selectively apply a force on the blood volume sensor and press the blood volume sensor onto the skin; a force sensor for measuring the force applied to the blood volume sensor and for outputting a force signal; and a control unit. The control unit is configured to receive a blood volume signal and a force signal for a time period in which the actuator increases the force applied on the blood volume sensor from a first force to a second force; determine a pulse pressure from the blood volume signal and the force signal, wherein the pulse pressure is the difference between a first blood pressure of the subject and a second blood pressure of the subject; process the blood volume signal to identify cardiac cycles and determine a blood volume amplitude for each cardiac cycle in the time period from the blood volume signal; determine, for each cardiac cycle, a ratio of the blood volume amplitude for the cardiac cycle and the determined pulse pressure; form a set of ratios from the determined ratios; and estimate the compliance of the blood vessel as a function of the set of ratios.

Fig. 1

EP 3 581 104 A1

**Description**

FIELD OF THE INVENTION

The disclosure relates to a method, device and computer program product for estimating a compliance of a blood vessel in a subject.

BACKGROUND OF THE INVENTION

Wearable on-body sensors are enabling better information about a subject's general health to be collected during their daily life. It is expected that such monitoring and analysis of the measurements, either automatically or by physicians, will reduce medical treatment costs, by preventing disease and enhancing quality of life. Wearable on-body sensors can be used to monitor vital signs such as heart rate, blood pressure, respiratory rate and core body temperature.

In the US about 30% of the adult population has a high blood pressure. Hypertension is a common health problem which has no obvious symptoms. Blood pressure generally rises with aging and the risk of becoming hypertensive in later life is considerable. Persistent hypertension is one of the key risk factors for strokes, heart failure and increased mortality. The condition of the subject can be improved by lifestyle changes, healthy dietary choices and medication. Particularly for high risk patients, continuous 24-hour blood pressure monitoring is very important by means of systems which do not impede ordinary daily life activities.

Blood pressure is defined by two measures: systolic and diastolic pressure. Systolic pressure occurs in the arteries during the maximal contraction of the left ventricle of the heart. Diastolic pressure refers to the pressure in arteries when the heart muscle is resting between beats and refilling with blood. Normal blood pressure is considered to be 120/80 mmHg. A person is considered to be hypertensive when the blood pressure is above 140/90 mmHg.

The ability of a blood vessel wall to expand and contract passively with changes in pressure is an important function of the cardiovascular system. The blood vessel compliance is the relationship between the volume of blood within a vascular segment and the blood pressure which is generated by the presence of that volume. For a given quantity of blood pressure, vessels with a high compliance can be filled with large volumes, while blood vessels having a low compliance can only be filled with small volumes. Arterial or vessel compliance $C_a$ is a measure of vascular volume change $\Delta V_a$ in response to a change in blood pressure $\Delta P$, i.e. $C_a = \Delta V_a / \Delta P$.

Various methods exist to estimate arterial compliance. Arterial stiffness assessed by means of oscillometry aims to describe the reduced capability of an artery to expand and contract in response to pressure changes induced by the pressure cuff. In the case of an oscillometric arm cuff, the stiffness of the brachial artery can be assessed by determining the relation between $\Delta V_a$ while simultaneously decreasing the pressure inside the cuff from above systolic pressure to diastolic pressure.

Arteriolosclerosis is a form of cardiovascular disease involving hardening and loss of elasticity of arterioles or small arteries and is most often associated with hypertension and diabetes mellitus. Arteriosclerosis occurs when the blood vessels that carry oxygen and nutrients from the heart to the rest of the body (arteries) become thick and stiff. This can result in a restriction of blood flow to organs and tissues. Healthy arteries are flexible and elastic. However, as time progresses, the walls of the arteries can become thicker, harder and less elastic, i.e. the compliance reduces. The compliance or stiffness in small arteries can be a marker for endothelial dysfunction that induces a functional change in the microcirculation of blood. The endothelium is a single layer of cells and acts as the ultimate layer between the blood vessel wall and the blood. The endothelium has many functions, including: serving as a (semi selective) barrier, preventing blood clotting, and enabling vasoconstriction and vasodilation. The endothelium can become damaged due to low density lipoprotein, chemicals from smoking and high blood pressure. Damaged endothelium cells may eventually lead to development of plaque onto the blood vessel wall, calcification of the blood vessel wall which stiffens the arteries, and formation of a blood clot. Endothelial dysfunction is a well-established response to cardiovascular risk factors and precedes the development of atherosclerosis. Endothelial dysfunction is usually ubiquitous throughout the body as patients with known atherosclerosis also have endothelial dysfunction in peripheral vascular beds.

Biological aging and atherosclerosis drive arterial stiffness. The major causes of arterial stiffness are vascular calcification, progressive degradation of artery elastin fibres and genetic factors. Increased arterial stiffness is associated with an increased risk of cardiovascular events such as myocardial infarction and stroke. Thus, arterial stiffness is regarded as an independent predictor of cardiovascular morbidity and mortality in hypertensive patients.

Therefore, it is desirable to be able to estimate the compliance of blood vessels in a subject in a straightforward and unobtrusive way.

SUMMARY OF THE INVENTION

According to a first specific aspect, there is provided a device for estimating a compliance of a blood vessel in a subject, the device comprising a blood volume sensor for measuring changes in blood volume in skin of the subject and for outputting a blood volume signal; an actuator arranged to selectively apply a force on the blood volume sensor and press the blood volume sensor onto the skin; a force sensor for measuring the force applied to the blood volume sensor and for outputting a force signal; and a control unit configured to: receive a blood volume signal and a force signal for a time period in which the actuator increases the force applied on the blood volume sensor from a first force to a second force; determine a pulse pressure from the blood volume signal and the force signal, wherein the pulse pressure is the difference between a first blood pressure of the subject and a second blood pressure of the subject; process the blood volume signal to identify cardiac cycles and determine a blood volume amplitude for each cardiac cycle in the time period from the blood volume signal; determine, for each cardiac cycle, a ratio of the blood volume amplitude for the cardiac cycle and the determined pulse pressure; form a set of ratios from the determined ratios; and estimate the compliance of the blood vessel as a function of the set of ratios.

In some embodiments, the control unit is further configured to control the actuator to apply force on the blood volume sensor.

In some embodiments, the control unit is further configured to control the actuator to substantially linearly increase the force applied on the blood volume sensor from the first force to the second force.

In some embodiments, the control unit is configured to determine a blood volume amplitude for each cardiac cycle in the time period from the blood volume signal by determining an AC component signal from the blood volume signal; processing the AC component signal to remove an AC component due to breathing by the subject; and applying a function to the processed AC component signal to estimate the blood volume amplitude for each cardiac cycle.

In some embodiments, the control unit is configured to estimate the compliance of the blood vessel as the mean of the ratios in the set of ratios.

In some embodiments, the control unit is further configured to estimate the first blood pressure and the second blood pressure for the subject from the blood volume signal and the force signal; and determine the pulse pressure from the estimated first blood pressure and the estimated second blood pressure of the subject.

In some embodiments, the first blood pressure is a lower blood pressure than the second blood pressure. In some embodiments, the first blood pressure is diastolic blood pressure. In some embodiments, the second blood pressure is systolic blood pressure.

In some embodiments, the control unit is configured to determine a ratio of blood volume amplitude and determined pulse pressure for any cardiac cycle where the determined blood volume amplitude is above a threshold.

In some embodiments, the control unit is configured to form the set of ratios from the ratios for cardiac cycles where the determined blood volume amplitude is above the threshold.

In some embodiments, the threshold is based on a blood volume amplitude measured when the actuator is applying the first force on the blood volume sensor.

In some embodiments, the blood vessel is an arteriole.

According to a second aspect, there is provided a method of estimating a compliance of a blood vessel in a subject using a device, the device comprising a blood volume sensor for measuring changes in blood volume in skin of the subject and for outputting a blood volume signal, an actuator arranged to selectively apply a force on the blood volume sensor and press the blood volume sensor onto the skin, and a force sensor for measuring the force applied to the blood volume sensor and for outputting a force signal, the method comprising: receiving a blood volume signal and a force signal for a time period in which the actuator increases the force applied on the blood volume sensor from a first force to a second force; determining a pulse pressure from the blood volume signal and the force signal, wherein the pulse pressure is the difference between a first blood pressure of the subject and a second blood pressure of the subject; processing the blood volume signal to identify cardiac cycles and determine a blood volume amplitude for each cardiac cycle in the time period from the blood volume signal; determining, for each cardiac cycle, a ratio of the blood volume amplitude for the cardiac cycle and the determined pulse pressure; forming a set of ratios from the determined ratios; and estimating the compliance of the blood vessel as a function of the set of ratios.

In some embodiments, the method further comprises the step of controlling the actuator to apply force on the blood volume sensor.

In some embodiments, the method further comprises controlling the actuator to substantially linearly increase the force applied on the blood volume sensor from the first force to the second force.

In some embodiments, the step of determining a blood volume amplitude for each cardiac cycle in the time period from the blood volume signal comprises: determining an AC component signal from the blood volume signal; processing the AC component signal to remove an AC component due to breathing by the subject; and applying a function to the processed AC component signal to estimate the blood volume amplitude for each cardiac cycle.

In some embodiments, the step of estimating the compliance of the blood vessel comprises estimating the compliance of the blood vessel as the mean of the ratios in the set of ratios.

In some embodiments, the method further comprises estimating the first diastolic blood pressure and the second blood pressure for the subject from the blood volume signal and the force signal; and determining the pulse pressure from the estimated first blood pressure and the estimated second blood pressure of the subject.

In some embodiments, the first blood pressure is a lower blood pressure than the second blood pressure. In some embodiments, the first blood pressure is diastolic blood pressure. In some embodiments, the second blood pressure is systolic blood pressure.

In some embodiments, the step of determining a ratio comprises determining a ratio of blood volume amplitude and determined pulse pressure for any cardiac cycle where the determined blood volume amplitude is above a threshold.

In some embodiments, the step of forming the set of ratios comprises forming the set from the ratios for cardiac cycles where the determined blood volume amplitude is above the threshold.

In some embodiments, the threshold is based on a blood volume amplitude measured when the actuator is applying the first force on the blood volume sensor.

In some embodiments, the blood vessel is an arteriole.

According to a third aspect, there is provided a computer program product comprising a computer readable medium having computer readable code embodied therein, the computer readable code being configured such that, on execution by a suitable computer or processor, the computer or processor is caused to perform the method according to the second aspect or any embodiment thereof.

In some embodiments, the suitable computer or processor has one or more inputs or interfaces to enable the computer or processor to receive the blood volume signal and the force signal. In some embodiments, the suitable computer or processor has one or more outputs to enable the computer or processor to control the actuator.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 shows schematically and exemplarily an embodiment of a device for estimating the compliance of a blood vessel of a subject;
Fig. 2 illustrates schematically and exemplarily a preferred position of the device while measuring a blood volume signal;
Fig. 3 shows a flow chart exemplarily illustrating an embodiment of a method for estimating the compliance of a blood vessel of a subject;
Fig. 4 shows exemplary Force-Stiffness curves for two subjects;
Fig. 5 shows a flow chart exemplarily illustrating an embodiment of a method for estimating the blood pressure of a subject;
Fig. 6 illustrates schematically and exemplarily a start of a diminishing of an amplitude of an oscillating component of a PPG signal in reaction to an increasing force;
Fig. 7 illustrates exemplarily assignments between force values at which an amplitude of an oscillating component of a PPG signal starts to diminish in reaction to an increasing force and diastolic arterial blood pressures; and
Fig. 8 shows exemplarily assignments between force values at which an amplitude of an oscillating component of a PPG signal becomes zero in reaction to an increasing force and systolic arterial blood pressures.

DETAILED DESCRIPTION OF EMBODIMENTS

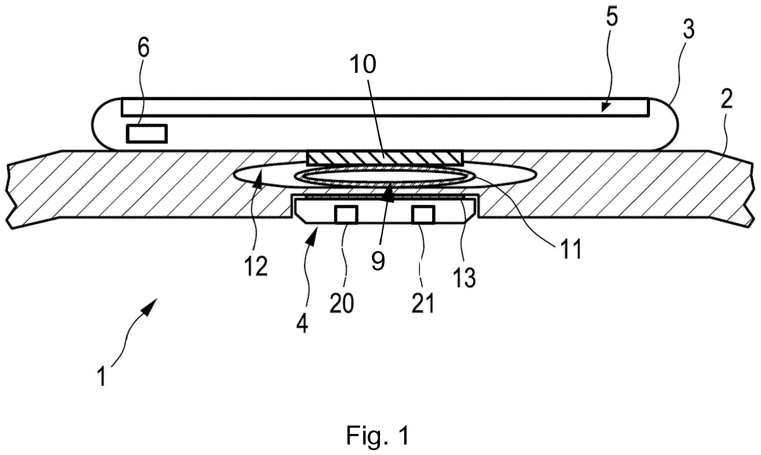

Fig. 1 shows schematically and exemplarily an embodiment of a device for estimating the compliance of a blood vessel of a subject. The estimated compliance is also referred to herein as 'blood vessel compliance' and 'blood vessel compliance index'. The subject can be a human or an animal. The device 1 comprises a blood volume sensor 4 for measuring changes in the blood volume in skin of the subject at a measurement location and for outputting a signal representing the blood volume over time or changes in the blood volume over time (referred to herein as a blood volume signal). The blood volume signal can be provided to a control unit 6 in the device 1. In some embodiments, the blood volume sensor 4 is a photoplethysmography (PPG) sensor for measuring a PPG signal of the skin and outputting a PPG signal. The PPG sensor 4 can be regarded as a blood pulse volumetric sensor, wherein the underlying concept is pulse oximetry which tracks the average skin tone which varies with the blood volume and blood oxygenation. The PPG sensor 4 comprises a light source 20, such as a light emitting diode (LED), which shines light on the skin. The light source 20 can emit green light to generate the PPG signal, and it will be understood that the wavelength of the emitted light will determine the depth in the skin at which the blood volume is measured, and thus determine the type of blood vessel that is being monitored. In other embodiments, the light source 20 can emit red and/or infrared light instead of, or in addition to, green light. In preferred embodiments, the wavelength of light is selected to measure the blood volume in capillaries and/or arterioles near the surface of the skin (e.g. a wavelength corresponding to green light). In other embodiments, the wavelength of light is selected to measure the blood volume in an artery (e.g. a wavelength corresponding to red or infrared light). The PPG sensor 4 further comprises a light sensor 21, such as a photodiode, for measuring the amount of light reflected by the skin. During each cardiac cycle (i.e. heart beat) the amount of reflected light varies as more or less blood flows through the arterioles/arteries/capillaries. The variation in skin tone over time contains information on the blood volume and the light sensor 21 converts the reflected light into an electrical signal, i.e. into the PPG signal (which is the blood volume signal).

A PPG signal consists of a DC component that is determined by the absorption of the non-pulsatile part of the tissue/skin and the constant volume of the pulsatile blood, and an AC component determined by the volume changes of blood vessels.

Those skilled in the art will be aware of other types of sensors that can be used to measure blood volume, for example an accelerometer, a camera-based PPG sensor, an ultrasound sensor, an inflatable cuff and pressure/volume sensor.

In the illustrated embodiment, the device 1 is a wrist-worn device including a flexible strap or wrist band 2 having two ends, wherein securement means are provided for circumferentially fastening the strap around the wrist. In this embodiment the blood volume sensor 4 is integrated in the wrist band 2. The blood volume sensor 4 is arranged within the device 1 such that it is in direct contact with the skin of the wrist of the user, when the device 1 is worn by the user. In alternative embodiments, the device 1 can be arranged or configured to be worn on a different part of the body of the subject, such as a finger, arm, chest, abdomen, leg, ankle, etc. It will be appreciated that in the wrist-worn or alternative embodiments above, attachment means other than a strap or band can be used to attach or hold the device 1 on the body of the subject.

The device 1 further comprises an actuator 9 for applying a force to the blood volume sensor 4 to press the blood volume sensor 4 into the skin of the subject. Thus, when the device 1 is being worn by a subject, the blood volume sensor 4 is located between the actuator 9 and the skin, and the actuator 9 can selectively apply force to the blood volume sensor 4 and the measurement location on the skin where the blood volume is measured. The actuator 9 therefore pushes the underlying blood volume sensor 4 onto the skin by exerting a force on the underlying blood volume sensor 4. The actuator 9 can be controlled by the control unit 6, and in particular the control unit 6 can control actuator 9 to selectively apply a force to the blood volume sensor 4, and for example control the actuator 9 to apply a first force, and to increase the applied force to a second force.

In the illustrated embodiment, the actuator 9, which might also be regarded as being a compression means, comprises a distensible or inflatable airbag 11 in a cavity 12 in the wrist band 2 and an air pump and valve combination 10 for inflating and deflating the airbag 11. In other embodiments, other types of actuator 9 can be used. For instance, an actuator 9 can be provided that is adapted to reduce the inner circumference of the wrist band 2, in order to apply an increasing force to the blood volume sensor 4 and the skin of the user's wrist.

The device 1 further comprises a force sensor 13 for measuring the force applied to the blood volume sensor 4 and for outputting a force signal representing the measured force over time. The force signal can be provided to the control unit 6. The force sensor 13 is arranged in between the actuator 9 and the blood volume sensor 4, and therefore the force measured by the force sensor 13 is a result of the pressing of the blood volume 4 into the skin by the actuator 9 and the resistance to compression of the skin/body part of the subject. The force sensor 13 can be, for instance, a force transducer, a strain gauge or a piezoelectric sensor. However, the force sensor 13 can also comprise other means for measuring the force applied to the skin.

In use of the device, the blood volume sensor 4 measures blood volume and the force sensor 13 measures force while the actuator 9 is operated to apply a varying (typically increasing) force to the blood volume sensor 4.

The control unit 6 is provided to control the operation of the device 1, and in particular to estimate the compliance of a blood vessel in the subject from the blood volume signal and the force signal. As such, the control unit 6 can be configured to execute or perform the methods described herein. The control unit 6 can be implemented in numerous ways, with software and/or hardware, to perform the various functions described herein. The control unit 6 may comprise one or more microprocessors or digital signal processor (DSPs) that may be programmed using software or computer program code to perform the required functions and/or to control components of the control unit 6 to effect the required functions. The control unit 6 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The control unit 6 may be connected to or comprise a memory unit (not shown) that can store data, information and/or signals for use by the control unit 6 in controlling the operation of the device 1 and/or in executing or performing the methods described herein. In some implementations the memory unit stores computer-readable code that can be executed by the control unit 6 so that the control unit 6 performs one or more functions, including the methods described herein. The memory unit can comprise any type of non-transitory machine-readable medium, such as cache or system memory including volatile and non-volatile computer memory such as random access memory (RAM) static RAM (SRAM), dynamic RAM (DRAM), read-only memory (ROM), programmable ROM (PROM), erasable PROM (EPROM) and electrically erasable PROM (EEPROM), implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In some embodiments, the control unit 6, or the functions of the control unit 6 in estimating the blood vessel compliance may be separate from the main part of the device 1 (i.e. separate from the blood volume sensor 4 and force sensor 13 that are worn on the wrist/body part). For example the control unit 6 or the functions of the control unit 6 may be performed by a computing device, such as a smart phone, tablet, laptop computer, etc., in which case the blood volume signal and the force signal can be provided to the computing device from the body-worn part of the device 1 by any suitable wired or wireless connection. It will be appreciated that in embodiments where the control unit 6 is part of a computing device, the computing device and the wrist-worn (or body-worn) part of the device are considered parts of the overall device 1.

In some embodiments, for example as shown in Fig. 1, the device 1 comprises a display screen 5 that enables the device 1 (and particularly the control unit 6) to output information or data to the subject, such as an estimate of the blood vessel compliance. The display screen 5 may be a touch screen that enables the subject to input information, data and/or commands into the device 1. In addition or alternatively to the touch screen, the device 1 can include one or more other user interface components for enabling the subject to input information, data and/or commands into the device 1, including but not limited to a keyboard, keypad, one or more buttons, switches or dials, a mouse, a track pad, a touchscreen, a stylus, a camera, a microphone, etc. In addition or alternatively to the display screen 5, the device 1 can include one or more other output component(s), including but not limited to one or more lights or light elements, one or more loudspeakers, a vibrating element, etc.

In the embodiment of Fig. 1, the device 1 includes a casing or housing 3 that is arranged in or on the wrist band 2, and the display screen 5 and control unit 6 are arranged in the casing or housing 3.

As noted above, the device 1 can be a wrist-worn device, and for example may be in the form of, or integrated into, a smart watch. Alternatively, the device 1 can be in the form of a bracelet, a sweat band, etc.

It will be appreciated that a practical implementation of a device 1 may include additional components to those shown in Fig. 1. For example the device 1 will typically also include a power supply, such as a battery.

It will also be appreciated that although in the above-described embodiments the different components of the device 1 are distributed in a certain way in the wrist band 2 and in the casing 3 attached to the wrist band 2, these components can also be distributed in a different way. In addition, it is also possible that a casing 3 or a wrist band 2 is not present, wherein in the latter case the casing 3 is held in place on the wrist by another attachment means. It is also possible that only a wrist band 2 is present, wherein all components are integrated in the wrist band 2.

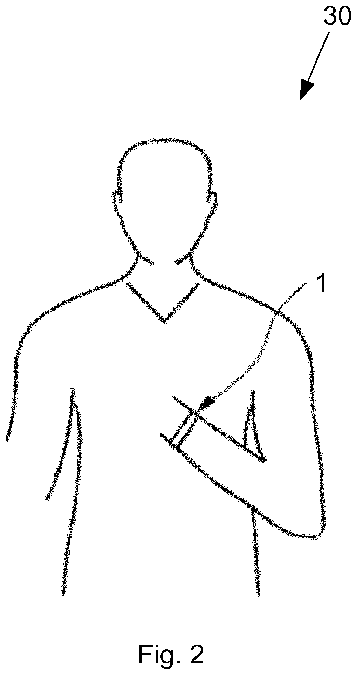

As noted below, the blood volume in a blood vessel (and thus the blood volume signal) can be influenced by a number of factors, other than blood vessel compliance. In some embodiments, one or more of these factors can be compensated for or taken into when estimating the blood vessel compliance. One factor is the breathing of the subject, and other factors include the posture (orthostatic pressure), arm position, obstruction of the arterial path if, for instance, the subject lies on their arm, and vasomotion. To remove or minimise the influence due to posture, i.e. the orthostatic pressure, the arm position and a possible obstruction of the arterial path, the measurement of the blood volume signal is preferentially standardised. Preferentially, the measurement of the blood volume signal is performed while the subject 30 is sitting or standing and the wrist is placed at heart height as schematically and exemplarily illustrated in Fig. 2.

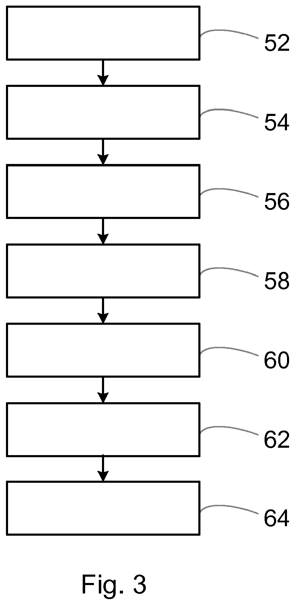

Fig. 3 shows a flowchart exemplarily illustrating an embodiment of a method for estimating the compliance of a blood vessel of a subject. The method in Fig. 3 can be implemented, performed and/or controlled by the control unit 6, for example in response to the control unit 6 executing suitable computer program code or computer program code portions that are stored in a memory unit or other transient or non-transient computer readable medium.

The method may be initiated by the subject, for example by putting the device 1 on, activating the device 1, or by initiating the compliance estimation procedure (e.g. by pressing a button). Alternatively the method can be initiated automatically by the control unit 6, for example in accordance with a schedule, or after a predetermined time has elapsed since a previous compliance estimation.

In step 52, the control unit 6 controls the actuator 9 to apply force to the blood volume sensor 4. In particular, in step 52 the control unit 6 controls the actuator 9 to increase a force applied to the blood volume sensor 4 from a first force to a second (maximum) force. The increase in the force applied is gradual, and preferably substantially linear or linear, and the increase from the first force to the second force takes a particular length of time. The first force can be zero (0) Newtons, N. The second force can be any suitable force, for example, a force between 60 mmHg and 280 mmHg. The rate of force increase from the first force to the second force (and thus the time taken to increase the force from the first force to the second force) depends on the resolution required for the measurement. For blood pressure, the accuracy may be within 5 mmHg. Since measurement points are only obtained during a heart beat, the system can increase the force between two heart beats. Consider two people, one with a heart rate of 60 beats/minute and another with a heart rate of 120 beats/minute, the force can be increased twice as fast for the second person. With a typical heart rate of 60 bpm and a maximum force increase of 5mmHg/second to a maximum force of 280 mmHg, the time taken to increase the force to the maximum will be 56 seconds. It will be appreciated that although the actuator 9 may initially apply no force to the blood volume sensor 4, the wrist band or strap 2 may itself hold the blood volume sensor 4 in place on the skin of the subject. In the embodiment shown in Fig. 1, step 52 comprises controlling the pump and valve combination 10 to inflate the airbag 11 enclosed in the cavity 12.

In step 54, which occurs while the force applied to the blood volume sensor 4 is increased from the first force to the second force in step 52, the blood volume sensor 4 and the force sensor 13 measure the blood volume and the force applied to the blood volume sensor 4 respectively to determine the blood volume signal and the force signal respectively. The blood volume signal and the force signal are provided to the control unit 6 for processing. Once the required blood volume signal and force signal have been acquired by the blood volume sensor 4 and force sensor 13 respectively, the actuator 9 can be controlled to reduce the applied force back to the first force, or some other force as required (e.g. by deflating the airbag 11 by opening a valve 10).

Steps 56-64 relate to the processing of the blood volume signal and the force signal to estimate the blood vessel compliance, and in particular the compliance of the blood vessel is estimated based on the blood volume signal and the force signal. It will be appreciated that this processing can be performed in real time or in near real time (e.g. as the blood volume signal and the force signal are measured or received by the control unit 6), or the processing can be performed some time later (for example when the subject or a care provider, such as a clinician, requires an estimate of the blood vessel compliance), in which case the blood volume signal and the force signal can be stored until the processing is to be performed.

In step 56, the control unit 6 determines a pulse pressure (denoted $\Delta P$) from the blood volume signal and the force signal. The pulse pressure is the difference between a first blood pressure and a second (higher) blood pressure. In some embodiments, the second blood pressure is the systolic blood pressure $P_s$. In some embodiments, the first blood pressure is the diastolic blood pressure $P_d$. Thus, in some embodiments, the pulse pressure is defined as: $\Delta P = P_s - P_d$. In some embodiments of step 56, the control unit 6 can estimate the second (e.g. systolic) and the first (e.g. diastolic) blood pressure of the subject from the blood volume signal and the force signal, and determine the pulse pressure from those estimates. Those skilled in the art will be aware of various ways in which a blood volume signal and a force signal can be processed to determine blood pressure. A specific, but exemplary, technique for determining diastolic and systolic blood pressure is described in more detail below. The systolic blood pressure is denoted $P_s$ and the diastolic blood pressure is denoted $P_d$.

In step 58, the control unit 6 determines a blood volume amplitude for each cardiac cycle covered by the blood volume signal. Thus, step 58 comprises processing the blood volume signal to identify cardiac cycles, and determining a blood volume amplitude for each cardiac cycle in the time period from the blood volume signal. The blood volume amplitude, denoted $V^i_a$, is determined for each ith cardiac cycle (i.e. each beat of the heart) during the time period in which the applied force increases from the first force (e.g. 0N) to the second force.

In some embodiments, step 58 comprises several sub-steps. Firstly, the blood volume signal can be processed to determine an AC component of the signal, and for example a respective AC component can be determined for each ith cardiac cycle (denoted $AC_i$). Cardiac cycles can be identified by using a beat detector algorithm. The algorithm has as input the blood volume signal (e.g. PPG signal). The algorithm aims to detect the beat onset moments in the blood volume signal. The interval between two consecutive beat onsets corresponds to a full cardiac cycle. Those skilled in the art will be aware of various techniques for determining or extracting an AC component of a signal. The extracted AC component of the blood volume signal for each cardiac cycle can be processed to remove an AC component due to breathing by the subject (since breathing influences blood volume). Again, those skilled in the art will be aware of various techniques for removing a breathing component from an AC component of a blood volume signal. For example, the AC components could be high-pass filtered in the frequency domain to remove the breathing-related component, since breathing has a lower frequency than heart beats. A particular technique is described in more detail below. In some embodiments, a second blood volume sensor (e.g. a PPG sensor) can be placed on the subject distal to the first blood volume sensor 4 described above (i.e. the second blood volume sensor is placed further away from the centre of the body of the subject than the first blood volume sensor 4). No additional force is applied to this second blood volume sensor, and breathing information is extracted from the second blood volume sensor measurement signal, and this information is used to eliminate the effect of breathing in the blood volume signal from the first blood volume sensor 4 (for example by subtracting the signal associated with the breathing component from the blood volume signal measured with the first blood volume sensor 4). In an alternative approach, the blood volume signal can be analysed to identify a signal envelope relating to breathing.

The resulting breathing-compensated $AC_i$ components are quasi-proportional to the blood volume changes in the compressed skin under the blood volume sensor 4. Finally, a function is applied to the breathing-compensated $AC_i$

components that translate the pulsatile $AC_i$ components into an estimate of the blood volume amplitude for each cardiac cycle i. The blood volume amplitude $V_a$ for each cardiac cycle can be given by: $V^i_a = f(ACi)$. The function may be, for example, a regression function that maps the AC estimate (possibly extended with additional predictors) into a 'true' blood volume amplitude.

In step 60, the control unit 6 determines a ratio of the determined pulse pressure and the blood volume amplitude for each cardiac cycle. In some embodiments, in step 60 the control unit 6 determines a ratio of the determined pulse pressure and the blood volume amplitude for each cardiac cycle where the blood volume amplitude is sufficiently large, i.e. the determined blood volume amplitude is above a threshold. The threshold is used to estimate the compliance of the blood vessel based on blood volume estimates in the situation where the monitored blood vessel has not been fully collapsed due to the externally applied force (i.e. the systolic pressure has not yet been reached when increasing the external force from the first force to the second (maximum) force).

The threshold used to identify a sufficiently large blood volume amplitude can be based on the estimated blood volume amplitude when no force is applied to the blood volume sensor 4 (e.g. the blood volume amplitude measured at the start of a measurement when the force applied by the actuator 9 equals the 'first force'). A blood volume amplitude $V'_a$, estimated for the ith cardiac cycle within the measurement period, is sufficiently large in the case that $V^i_a$ satisfies: $V^i_a$ > threshold, where the threshold = $V^{i=1}a$ * k, and k = 0.05. $V^{i=1}a$ refers to the estimated blood volume amplitude of the first cardiac cycle $i=1$, i.e. at the start of the measurement period, where the externally applied force by the actuator 9 equals the 'first force' (e.g. 0 N). The value set for the coefficient k can be an arbitrary choice, and can be adjusted to increase/decrease the sensitivity of the exclusion criterion.

In step 62, each such ratio (e.g. the ratio for any cardiac cycle where the blood volume amplitude is above the threshold) is included in a set of ratios. Thus, step 60 can comprise comparing each of the blood volume amplitude for each of the $i$ cardiac cycles to the threshold, and for any blood volume amplitude above the threshold, a ratio of that blood volume amplitude over the pulse pressure is determined. Thus, the ratio for the ith cardiac cycle is denoted $s_i$ and is given by $s_i = V^i_a / \Delta P$, giving a set $S = \{s_i,...,s_N\}$ (in step 62), where N is the number of blood volume amplitudes that are above the threshold. It will be appreciated that each ratio is a measure of the compliance of the blood vessel in the respective cardiac cycle.

Finally, in step 64, the control unit 6 determines an estimate of the compliance of a blood vessel from the set of ratios. In particular, the control unit 6 determines the estimate of the compliance of a blood vessel as a function of the set of ratios. The compliance $C_a$ can be inferred from the set S by means of a function such as the arithmetic mean of the set S. In other words, in this example, the overall compliance of the blood vessel is determined as the mean compliance estimated from the various cardiac cycles where the blood volume amplitude is above the threshold. Other functions of the set of ratios could be used to determine the overall compliance $C_a$. For example, the overall compliance $C_a$ could be given as the mode or median of the ratios in set S, the maximum ratio $s_i$ in the set S, etc. Other functions that can be used include, for example, functions that model the ratio time series by fitting a higher order polynomial function. The coefficients of the model can be used to compute/infer the overal compliance.

In some embodiments, the method can further comprise a step of outputting the compliance estimate. The compliance estimate can be output on a display screen 5 (if present in the device 1), and/or the compliance estimate can be output by the control unit 6 by some other means (e.g. a loudspeaker) and/or output to another device, for example a computer or computing device.

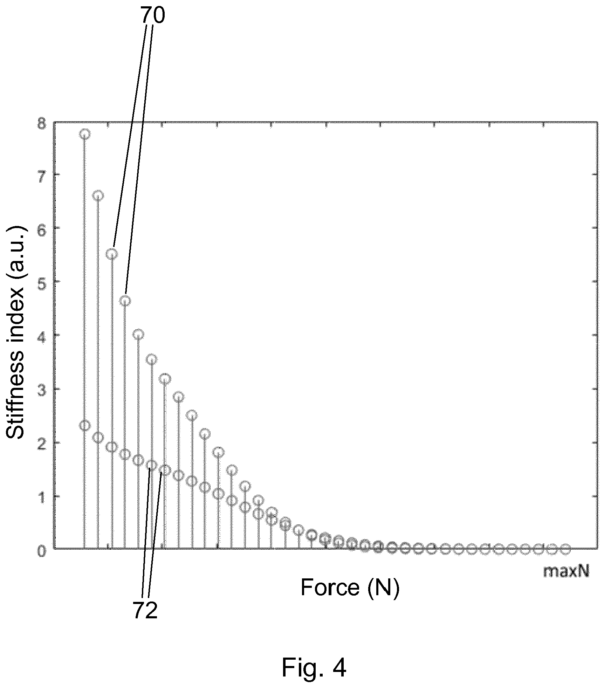

A relationship between applied external force (i.e. by actuator 9), which generally linearly increases from a first force (e.g. 0N) to a maximum force (maxN), and the resulting compliance estimated per individual cardiac cycle, can be visualised in a Force-Stiffness index curve. Two exemplary Force-Stiffness index curves are shown in Fig. 4 for a subject with high compliance/elastic arterioles (represented by data points 70) and another subject with a large degree of stiffness in the probed arterioles (represented by data points 72), in case the pulse pressure $\Delta P$ is the same for both subjects. It can be seen that, particularly at low applied forces, the more compliant blood vessel induces higher blood volume amplitudes than the stiffer blood vessel. Fig. 4 effectively shows the derived individual compliance indices (i.e. for each cardiac cycle) that constitute or define the set S. The overall compliance of the blood vessel can be inferred from the set S, as in step 64 of Fig. 3.

Estimating diastolic and systolic blood pressure

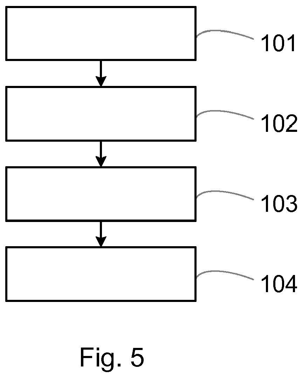

The section describes an exemplary technique for estimating the diastolic blood pressure in step 56 of Fig. 3, and is described for an embodiment where the blood volume sensor 4 is a PPG sensor and the blood volume signal is a PPG signal. Briefly, with reference to the flow chart in Fig. 5, an oscillating component of the PPG signal is determined by high-pass filtering the PPG signal (step 101), a force value of the increasing force at which the amplitude of the oscillating component of the PPG signal starts to diminish in reaction to the increasing force (applied by the actuator 9) is determined (step 102), assignments between force values at which an amplitude of an oscillating component of a PPG signal starts to diminish in reaction to the increasing force and diastolic arterial blood pressures are provided (step 103), and the diastolic arterial blood pressure is determined based on the determined force value and the provided assignments (step 104).

In step 101, a cut-off frequency of 0.5 Hz can be used for high-pass filtering the PPG signal to determine the oscillating component. The oscillating component will be the pulsatile component of the PPG signal.

In step 102, the force value of the increasing force at which the amplitude of the oscillating component of the PPG signal starts to diminish in reaction to the increasing force can be determined by only considering changes of the amplitude of the oscillating component which are larger than a predefined threshold. The changes are preferentially changes with respect to consecutive amplitudes of the oscillating component. If changes are not considered that are not larger than the predefined threshold, it can be prevented that fluctuations in the amplitude of the oscillating component of the PPG signal that are not related to the application of the increasing force lead to a wrong determination of the force value of the increasing force at which the amplitude of the oscillating component of the PPG signal starts to diminish in reaction to the increasing force. The predefined threshold can be determined by, for instance, calibration measurements during which the increasing force is not applied and changes of the amplitude of the oscillating component are determined, wherein these changes are than not caused by the increasing force and can define the threshold.

Moreover, the force value can be determined in step 102 such that it is the force value of the increasing force at which the amplitude of the oscillating component of the PPG signal starts to monotonically diminish in reaction to the increasing force.

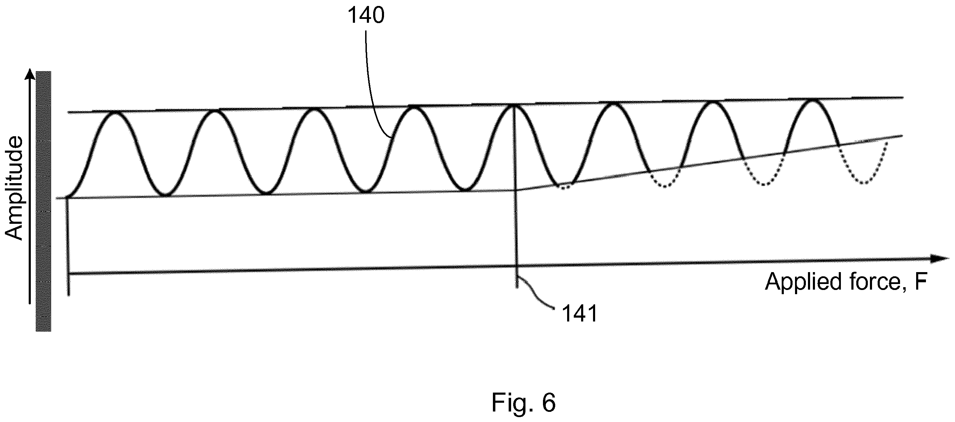

Fig. 6 illustrates the start of the diminishing of an amplitude of an oscillating component of a PPG signal in reaction to an increasing force. In Fig. 6 it can be seen that the amplitude of the oscillating component of the PPG signal 140 is firstly constant, although the applied force F is increased, wherein at a force value indicated by the line 141 the amplitude of the oscillating component of the PPG signal 140 starts to diminish. At this force value 141 the external pressure applied on the arterioles exceeds the diastolic pressure. Step 102 aims to identify the force value corresponding to line 141.

Furthermore, before determining the force value in step 102, the amplitude of the oscillating component of the PPG signal can be corrected for breathing influences. As such, an indicator of the breathing of the subject is required, and this breathing indicator can be used to determine the breathing influences and to correct the oscillating component of the PPG signal for these determined breathing influences. For instance, step 102 can comprise modelling the breathing influences based on the provided breathing indicator and to use the modelled breathing influences to correct the amplitude of the oscillating component of the PPG signal which has been measured while the force applied to the skin was increased. The breathing indicator is preferentially a value or a signal which changes over time in accordance with the subject's breathing. The breathing indicator can be, for instance, provided by an accelerometer which provides an accelerometer signal in accordance with the breathing, or by a breathing belt. The breathing indicator is preferentially measured at or preferentially corresponds to a time before the increasing force is applied to the skin by the actuator 9, in order to obtain a breathing indicator which is less likely corrupted by the increasing applied force. The breathing indicator can also be obtained by processing a signal measured by a sensor that measures the heart rate and/or the inter-beat-interval. For deriving the breathing indicator from a measurement of the heart rate and/or the inter-beat-interval known techniques can be used like the techniques disclosed in the article *"Developing an algorithm for pulse oximetry derived respiratory rate (RRoxi): a healthy volunteer study"*, Paul S. Addison et al., volume 26, pages 45 to 51, Journal of Clinical Monitoring and Computing (2012), which is herewith incorporated by reference.

Besides breathing, the oscillating component of the PPG signal can be influenced by other factors like posture (orthostatic pressure), arm position, obstruction of the arterial path if, for instance, the subject lies on their arm, and vasomotion. In order to control the posture, i.e. the orthostatic pressure, the arm position and a possible obstruction of the arterial path, the measurement of the PPG signal and also of the breathing indicator is preferentially standardised. Preferentially, the measurement of the PPG signal and also of the breathing indicator is carried out, while the subject 30 is sitting or standing and the wrist is placed at heart height as schematically and exemplarily illustrated in Fig. 2.

A further (second) PPG sensor can be provided for measuring a further PPG signal, wherein this further PPG signal can be used for determining the breathing behaviour, i.e. for determining the breathing indicator being indicative of the breathing behaviour. This further PPG signal used for determining the breathing behaviour can be measured before applying the force to the skin or, if the force application does not influence the further PPG signal, while the force is applied to the skin. It is also possible that the (first) PPG sensor measures a PPG signal before the increasing force is applied to the skin, the breathing influences can be determined based on this PPG signal and these determined breathing influences are used for correcting the amplitude of the oscillating component of the PPG signal which is then measured while the force applied to the skin is increased. In this case any further sensor, i.e. a breathing sensor, for measuring a signal from which the breathing behaviour can be derived is preferentially not present. In particular, since the breathing rate is normally about 12 breaths per minute, before applying the increasing force to the skin and measuring the actual PPG signal which will be used for determining the force value of the increasing force at which the amplitude of the oscillating component of this PPG signal starts to diminish, a PPG signal can be measured over a time period of two full cycles or more, i.e., for instance, over 10 seconds or more, in order to provide enough information for assessing how the oscillating component of the PPG signal behaves due to breathing, wherein this information can be used to model the influence of the applied force on top of that.

For instance, the breathing rate can be determined by using the high-pass filtered PPG signal, i.e. the oscillating component, or the non-filtered PPG signal before applying the increasing force and/or when the application of the increasing force just started. For determining the breathing rate it is possible to, for instance, use the technique disclosed in the above mentioned article by Paul Addison, which is based on detecting a DC variation, an AC variation and an R-R variation, or to use other known techniques. For the modelling of the respiratory behaviour of the high-pass filtered PPG signal the determined breathing rate can be extrapolated or it can be assumed that the R-R variation continues.

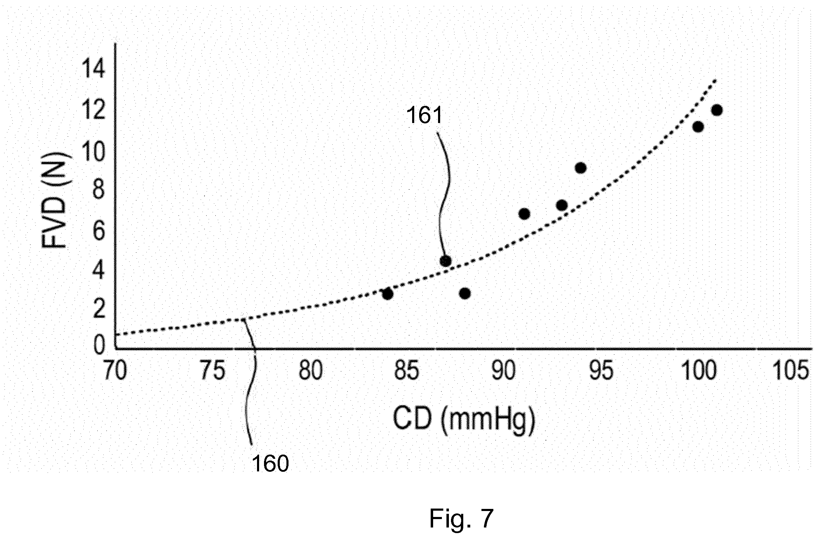

In step 103, as noted above, assignments between force values at which an amplitude of an oscillating component of a PPG signal starts to diminish in reaction to an increasing force and diastolic arterial blood pressures are provided. The assignments can be generated in a training or calibration procedure, wherein the device 1 is used for determining force values of the increasing force for different subjects at which the amplitude of the oscillating component of the PPG signal starts to diminish in reaction to the increasing force, wherein the diastolic arterial blood pressure is known from measurements with a blood pressure cuff. Such measurements are illustrated in Fig. 7 showing the force values (FVD) of the increasing force at which the amplitude of the oscillating component of the PPG signal starts to diminish in reaction to the increasing force versus the diastolic arterial blood pressure (CD) which has been measured by using a blood pressure cuff. In Fig. 7 the points 161 are measurement points and the line 160 is an exponential fit through the points. In this example the assignments between force values FVD and diastolic arterial blood pressures CD are provided by the line 160.

Finally, in step 104, the diastolic arterial blood pressure is determined based on the determined force value and the assignments 160. The method in Fig. 5 thus allows for a determination of diastolic arterial blood pressure from locally induced pressure on the skin.

The blood flow Q, when passing through a vessel that is too small to allow all blood to flow freely, is directly related to blood pressure P through the resistance of the blood vessel $R$, expressed in the following physical relationship:

$$Q = \frac{P_1 - P_2}{R} \tag{1}$$

where $P_1$ is the pressure at the start point of the vessel and $P_2$ is the pressure at the end point of the vessel.

This means that at a certain location within a blood vessel, the blood flow through is determined by the pressure difference before and after the location. However, the resistance in the location serves as a mediator: when $P_1 - P_2$ is kept constant but $R$ is increased, $Q$ will decrease proportional to $R$. The resistance of the vessel can be expressed in terms of the vessel properties as follows:

$$R = \frac{8L\eta}{\pi r^4} \tag{2}$$

where $L$ is the vessel length, $\eta$ is the blood viscosity and $r$ is the vessel radius. Thus:

$$Q = \frac{(P_1 - P_2)\pi r^4}{8L\eta} \tag{3}$$

Assuming blood viscosity to be constant, the vessel radius has a polynomial relationship with blood flow.

When $r$ is decreased with a known amount and the resulting decrease in $Q$ is measured, $P_1 - P_2$ can be computed after applying a multiplier c that takes into account the remaining factors:

$$Q = (P_1 - P_2)r^4 \cdot \frac{\pi}{8L\eta} = c(P_1 - P_2)r^4 \tag{4}$$

It is known that $P_1 - P_2$, the differential between pressure before and after the vessel, is not a constant but continuously modulated by the pulsating heart, which propagates a blood pulse with high pressure (i.e. systolic pressure) through the arterial system. However, even without the direct blood pulse (i.e. during diastole) there is a continuous pressure referred to as diastolic pressure that keeps blood circulating.

The PPG measurement is determined by the sum of absorptions from venous blood at very low pressure and arterial blood which increases at systole and decreases during diastole. Next to that the signal is also affected by non-blood tissue which should not be affected by pressure changes. Upon applying pressure on the skin, the following will happen. Firstly, venous blood volume will start to decrease which is expressed in the PPG signal as an increase in the baseline of the signal (overall higher reflectivity). After that, the arterial resistance will be high enough to affect the lowest level of diastolic pressure. At this point the pulsatile component of the PPG will start to shrink. This property is preferentially used by the control unit 6 for determining the diastolic arterial blood pressure. As the pressure on the skin is further increased, eventually the vessel resistance will be high enough to stop systolic pressure. At this point the pulse will be completely diminished and the PPG will become a non-pulsatile signal. This property is preferentially used by the control unit 6 for determining the systolic arterial blood pressure as will be explained in the following.

Preferably, the method of Fig. 5 also includes a step of determining a force value of the increasing force at which the amplitude of the oscillating component of the PPG signal becomes zero, in order to determine the systolic pressure. Correspondingly, assignments are provided between force values at which the amplitude of the oscillating component of the PPG signal becomes zero and systolic arterial blood pressures. The systolic arterial blood pressure is determined based on the determined force value at which the amplitude of the oscillating component of the PPG signal becomes zero and the provided assignments between force values at which the amplitude of the oscillating component of the PPG signal becomes zero and systolic arterial blood pressures.

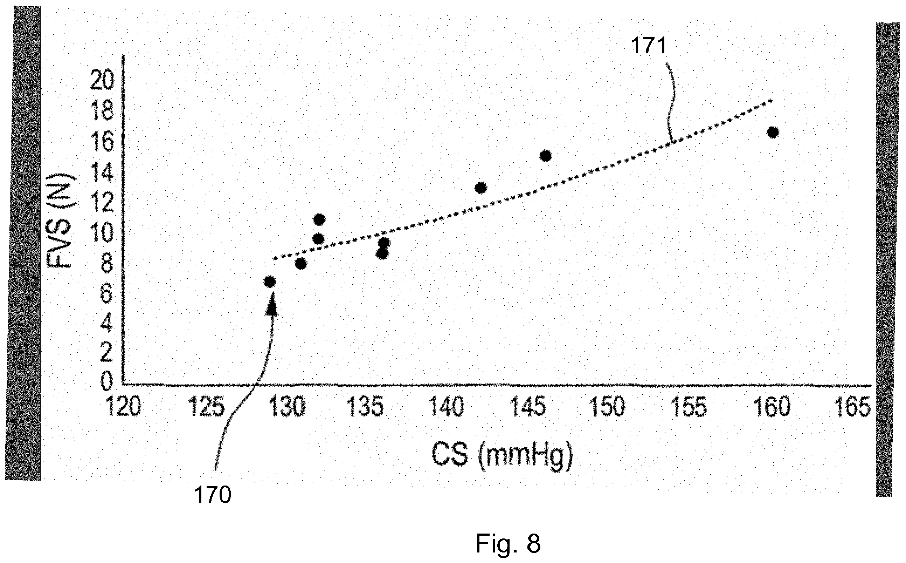

The assignments between the force values (FVS), at which the amplitude of the oscillating component of the PPG signal becomes zero and the systolic arterial blood pressures (CS) can be determined in a calibration or training procedure, wherein these force values FVS are determined while the systolic arterial blood pressures CS are known from, for instance, blood cuff measurements. Fig. 8 schematically and exemplarily illustrates such measurements 170 and a fitted line 171, wherein the fitted line 171 can be provided as the assignments to be used for determining the systolic arterial blood pressure.

Therefore, there is provided a method, device and computer program product for estimating a compliance of a blood vessel in a subject. The method, device and computer program product provide a straightforward and unobtrusive way to estimate compliance since the subject is not required to take any specific action in order to estimate the compliance (other than perhaps putting the relevant arm in a correct posture with respect to the heart), and the force applied during the measurement procedure is not significant, so discomfort for the subject is minimised. In some cases the compliance could be measured while the subject is asleep. The estimated compliance can be used as a marker or indicator for endothelial dysfunction and/or to assess the effects of antihypertensive medicines in small blood vessels (microvasculature, e.g. arterioles) in hypertension management applications.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A device for estimating a compliance of a blood vessel in a subject, the device comprising:

   a blood volume sensor for measuring changes in blood volume in skin of the subject and for outputting a blood volume signal;
   an actuator arranged to selectively apply a force on the blood volume sensor and press the blood volume sensor onto the skin;
   a force sensor for measuring the force applied to the blood volume sensor and for outputting a force signal; and
   a control unit configured to:

   receive a blood volume signal and a force signal for a time period in which the actuator increases the force applied on the blood volume sensor from a first force to a second force;
   determine a pulse pressure from the blood volume signal and the force signal, wherein the pulse pressure is the difference between a first blood pressure of the subject and a second blood pressure of the subject;
   process the blood volume signal to identify cardiac cycles and determine a blood volume amplitude for each cardiac cycle in the time period from the blood volume signal;

determine, for each cardiac cycle, a ratio of the blood volume amplitude for the cardiac cycle and the determined pulse pressure;

form a set of ratios from the determined ratios; and

estimate the compliance of the blood vessel as a function of the set of ratios.

2. A device as claimed in claim 1, wherein the control unit is further configured to control the actuator to apply force on the blood volume sensor.

3. A device as claimed in claim 2, wherein the control unit is further configured to control the actuator to substantially linearly increase the force applied on the blood volume sensor from the first force to the second force.

4. A device as claimed in any of claims 1-3, wherein the control unit is configured to determine a blood volume amplitude for each cardiac cycle in the time period from the blood volume signal by:

determining an AC component signal from the blood volume signal;

processing the AC component signal to remove an AC component due to breathing by the subject; and

applying a function to the processed AC component signal to estimate the blood volume amplitude for each cardiac cycle.

5. A device as claimed in any of claims 1-4, wherein the control unit is configured to estimate the compliance of the blood vessel as the mean of the ratios in the set of ratios.

6. A device as claimed in any of claims 1-5, wherein the control unit is further configured to:

estimate the first blood pressure and the second blood pressure for the subject from the blood volume signal and the force signal; and

determine the pulse pressure from the estimated first blood pressure and the estimated second blood pressure of the subject.

7. A device as claimed in any of claims 1-6, wherein the control unit is configured to form the set of ratios from the ratios for cardiac cycles where the determined blood volume amplitude is above a threshold.

8. A method of estimating a compliance of a blood vessel in a subject using a device, the device comprising a blood volume sensor for measuring changes in blood volume in skin of the subject and for outputting a blood volume signal, an actuator arranged to selectively apply a force on the blood volume sensor and press the blood volume sensor onto the skin, and a force sensor for measuring the force applied to the blood volume sensor and for outputting a force signal, the method comprising:

receiving a blood volume signal and a force signal for a time period in which the actuator increases the force applied on the blood volume sensor from a first force to a second force;

determining a pulse pressure from the blood volume signal and the force signal, wherein the pulse pressure is the difference between a first blood pressure of the subject and a second blood pressure of the subject;

processing the blood volume signal to identify cardiac cycles and determine a blood volume amplitude for each cardiac cycle in the time period from the blood volume signal;

determining, for each cardiac cycle, a ratio of the blood volume amplitude for the cardiac cycle and the determined pulse pressure;

forming a set of ratios from the determined ratios; and

estimating the compliance of the blood vessel as a function of the set of ratios.

9. A method as claimed in claim 8, wherein the method further comprises the step of controlling the actuator to apply force on the blood volume sensor.

10. A method as claimed in claim 9, wherein the method further comprises controlling the actuator to substantially linearly increase the force applied on the blood volume sensor from the first force to the second force.

11. A method as claimed in any of claims 8-10, wherein the step of determining a blood volume amplitude for each cardiac cycle in the time period from the blood volume signal comprises:

determining an AC component signal from the blood volume signal;

processing the AC component signal to remove an AC component due to breathing by the subject; and

applying a function to the processed AC component signal to estimate the blood volume amplitude for each cardiac cycle.

12. A method as claimed in any of claims 8-11, wherein the step of estimating the compliance of the blood vessel comprises estimating the compliance of the blood vessel as the mean of the ratios in the set of ratios.

13. A method as claimed in any of claims 8-12, wherein the method further comprises:

estimating the first diastolic blood pressure and the second blood pressure for the subject from the blood volume signal and the force signal; and

determining the pulse pressure from the estimated first blood pressure and the estimated second blood pressure of the subject.

14. A method as claimed in any of claims 7-13, wherein the step of forming the ratios comprises forming the set of ratios from the ratios for cardiac cycles where the determined blood volume amplitude is above a threshold.

15. A computer program product comprising a computer readable medium having computer readable code embodied therein, the computer readable code being configured such that, on execution by a suitable computer or processor, the computer or processor is caused to perform the method of any of claims 8-14.

Fig. 1

EP 3 581 104 A1

30

1

Fig. 2

Fig. 3

Fig. 4

Fig. 5

Fig. 6

EP 3 581 104 A1

Fig. 7

Fig. 8

EP 3 581 104 A1

Europäisches
Patentamt

European
Patent Office

Office européen
des brevets

# EUROPEAN SEARCH REPORT

Application Number

EP 18 17 7438

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/265166 A1 (TANAKA GOHICHI [JP]) 24 September 2015 (2015-09-24) * paragraph [0011] * * paragraph [0018] * * paragraph [0079] * * paragraph [0087] - paragraph [0089] * * paragraph [0112] * * paragraph [0147] - paragraph [0151] * * paragraph [0172] *<br>----- | 1-15 | INV. A61B5/022 A61B5/00 A61B5/021 A61B5/08 A61B5/113 A61B5/02 |
| X | WO 2017/216268 A1 (KONINKLIJKE PHILIPS NV [NL]) 21 December 2017 (2017-12-21) * abstract * * page 5, line 9 - line 15 * * page 6, line 1 - line 11 * * page 6, line 17 - line 19 * * page 11, line 24 - line 27 * * page 12, line 14 - line 15 *<br>----- | 1-15 | |
| A | WO 2017/152098 A1 (UNIV MICHIGAN STATE [US]; UNIV OF MARYLAND [US]) 8 September 2017 (2017-09-08) * paragraph [0058] - paragraph [0061] * * paragraph [0075] - paragraph [0083] * * paragraph [0110] - paragraph [0111] * * paragraph [0126] *<br>----- | 1-15 | TECHNICAL FIELDS SEARCHED (IPC)<br>A61B |

The present search report has been drawn up for all claims

| Place of search | Date of completion of the search | Examiner |
|---|---|---|
| The Hague | 13 November 2018 | Gooding Arango, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 17 7438

This annex lists the patent family members relating to the patent documents cited in the above-mentioned European search report.
The members are as contained in the European Patent Office EDP file on
The European Patent Office is in no way liable for these particulars which are merely given for the purpose of information.

13-11-2018

| Patent document cited in search report | Publication date | Patent family member(s) | Publication date |
|---|---|---|---|
| US 2015265166 A1 | 24-09-2015 | EP 2904968 A1<br>JP 6203737 B2<br>JP WO2014054788 A1<br>US 2015265166 A1<br>WO 2014054788 A1 | 12-08-2015<br>27-09-2017<br>25-08-2016<br>24-09-2015<br>10-04-2014 |
| WO 2017216268 A1 | 21-12-2017 | NONE | |
| WO 2017152098 A1 | 08-09-2017 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PAUL S. ADDISON et al.** *Journal of Clinical Monitoring and Computing,* 2012, vol. 26, 45-51